# EUROPÄISCHE PATENTANMELDUNG

(11) **EP 0 832 855 A2**
(43) Veröffentlichungstag der Anmeldung: **01.04.1998**
(21) Anmeldenummer: 97116671.5
(22) Anmeldetag: 24.09.1997
(51) Int. Cl.: C02F 11/04, C02F 11/14, C02F 1/42

(54) **Verfahren zum Vergären von Klärschlamm**

(30) Priorität: 26.09.1996 DE 19639612; 28.01.1997 DE 19703018
(71) Anmelder: FRAUNHOFER-GESELLSCHAFT ZUR FÖRDERUNG DER ANGEWANDTEN FORSCHUNG E.V., 80636 München (DE)
(72) Erfinder: Schmid-Staiger, Ulrike, Dr., 72654 Neckartenzlingen (DE); Schäfer, Joachim, 70178 Stuttgart (DE); Trösch, Walter, Dr., 70329 Stuttgart (DE)
(74) Vertreter: Olgemöller, Luitgard, Dr.

(57) **Zusammenfassung**

Die Erfindung betrifft ein Verfahren zum anaeroben Vergären von Klärschlamm, dadurch gekennzeichnet, daß der Klärschlamm physikalisch-chemisch entflockt wird. Dies kann dadurch erfolgen, daß dem Klärschlamm flockenbildende Ionen entzogen werden, beispielsweise durch Behandeln des Klärschlamms mit Ionenaustauscher oder durch Säure.

## Beschreibung

In der kommunalen Abwasserreinigung wird mit Hilfe von aeroben Mikroorganismen das verschmutzte Abwasser gereinigt. Ein wesentlicher Nachteil dieses Verfahrens ist die Tatsache, daß nur ca. 50% der Schmutzfracht in CO₂ überführt werden; die restlichen 50% werden in aerobe Biomasse umgesetzt. Somit kommt es zu einer Überschußproduktion von aerober Biomasse, die zusammen mit den nicht abgebauten Schmutzpartikeln in Form von Flocken aus dem System ausgetragen wird. Dieser Stoffstrom wird als Klärschlamm bezeichnet, der ein weiteres Entsorgungsproblem darstellt. Bei der aeroben Abwasserreinigung kommt es somit neben dem Reinigungseffekt zu einer Problemverlagerung von Abwasser zu Klärschlamm.

Mögliche Entsorgungswege für den Klärschlamm sind vor allem die Verbrennung, Deponierung, Ausbringung in die Landwirtschaft und die Vergärung (anaerober Abbau). Die Vergärung stellt nicht zuletzt wegen des Gewinns von Methan (einem regenerativen Energieträger) die nachhaltigste Variante dar. Eine Vielzahl von Forschungseinrichtungen beschäftigt sich daher mit der biologisch-technischen bzw. der verfahrenstechnischen Optimierung dieses Prozesses.

Dabei werden bisher maximal 50 - 55 % der Organik des Klärschlamms anaerob zu Biogas (Methan und CO₂) abgebaut. Theoretisch sind organische Substanzen aber bis zu 90-95 % zu Biogas umsetzbar. Die Werte für die Vergärung anderer organischer Substrate (z.B. Biomüll) bestätigen diese Zahl. Der in den Faultürmen unvollständig ablaufende Abbau wird in der Literatur im wesentlichen damit erklärt, daß nur ein Teil der aeroben Biomasse im anaeroben Milieu lysiert und abgebaut wird. Es wird allgemein davon ausgegangen, daß ein Teil der aeroben Biomasse im anaeroben Milieu des Faulturms überlebt (fakultative Anaerobier) oder zumindest nicht lysiert werden kann.

Aus diesem Grund beschäftigen sich eine Vielzahl von Forschergruppen mit der Entwicklung von Zellaufschlußverfahren.

Es herrscht die allgemeine Meinung, daß mit solchen Verfahren im Rahmen einer Schlammvorbehandlung alle aus der aeroben Stufe ausgetragenen Zellen aufgeschlossen werden und somit der Abbaugrad in der anaeroben Stufe gesteigert werden kann. Es werden mechanische Verfahren mit Hilfe einer Kugelmühle oder von Ultraschall, thermische Verfahren, enzymatische und chemische Vorbehandlungen vorgeschlagen. Trotz dieser Vorbehandlung des Schlamms (mit dem Ziel Zellaufschluß) erzielen auch diese Forschergruppen nur einen Umsatz der organischen Bestandteile von ca. 50%.

Auch die Erfinder haben sich bereits mit Zellaufschlußverfahren beschäftigt. Bereits stabilisierter Schlamm wurde aufkonzentriert, mit den oben beschriebenen Zellaufschlußverfahren behandelt und erneut der Vergärung zugeführt. Dabei wurden die bisherigen Ergebnisse bestätigt. Durch diese Vorgehensweise konnte der Abbaugrad nicht gesteigert werden.

Aufgabe der Erfindung ist es, bei der Schlammvergärung eine verbesserte Leistung zu erhalten. Hierdurch soll sowohl die Menge an Deponieschlamm verringert werden als auch die Gewinnung von Energie (in Form von Methan) gesteigert werden. Die Aufgabe wird gelöst durch ein Verfahren gemäß Anspruch 1 und bevorzugt nach einem der Ansprüche 2 bis 5. Die Ansprüche 6 bis 9 zeigen spezielle Ausgestaltungen der Erfindung.

Um die eigentliche Vergärbarkeit von Belebtschlammzellen zu untersuchen, ist in ideal durchmischten aeroben Bioreaktoren submers verteilter Belebtschlamm gezüchtet worden. Bei der hier angewandten Technik wurde die Ausbildung von Flockenstrukturen unterbunden. Überraschenderweise ließen sich die submers verteilten Belebtschlammzellen in anschließenden Vergärungsexperimenten sehr gut (> 80%) abbauen. Durch Zugabe von Ionen und Flockungsmitteln konnte eine Flockenbildung auch bei submersen Zellen erzielt werden. Bei der Vergärung dieser Belebtschlammflocken ist der Umsatz zu Biogas wieder zurückgegangen.

Diese Untersuchungen ließen den Schluß zu, daß die Abbaurate nicht vom Vorhandensein intakter Zellen abhängig ist. Im übrigen wurde auch festgestellt, daß es sich bei der Restorganik eines vergorenen Schlammes im wesentlichen gar nicht um übrigbleibende aerobe Zellen handelt. Untersuchungen ergaben vielmehr, daß Proteine, Kohlenhydrate und ein häufig großer Anteil an Huminstoffen (bis ca. 50%) vorhanden ist. Bekanntlich fungieren Huminstoffe als Komplexbildner für mehrwertige Ionen, so daß gerade diese Stoffe einen beträchtlichen Anteil am Entstehen der Flocken haben dürften. Dabei steigen die Bindungskräfte zwischen Huminstoffen und Ionen mit deren Wertigkeit. Daher stellt wahrscheinlich die genannte, häufig kompakte Flockenstruktur den Schlüssel für den begrenzten Abbau dar.

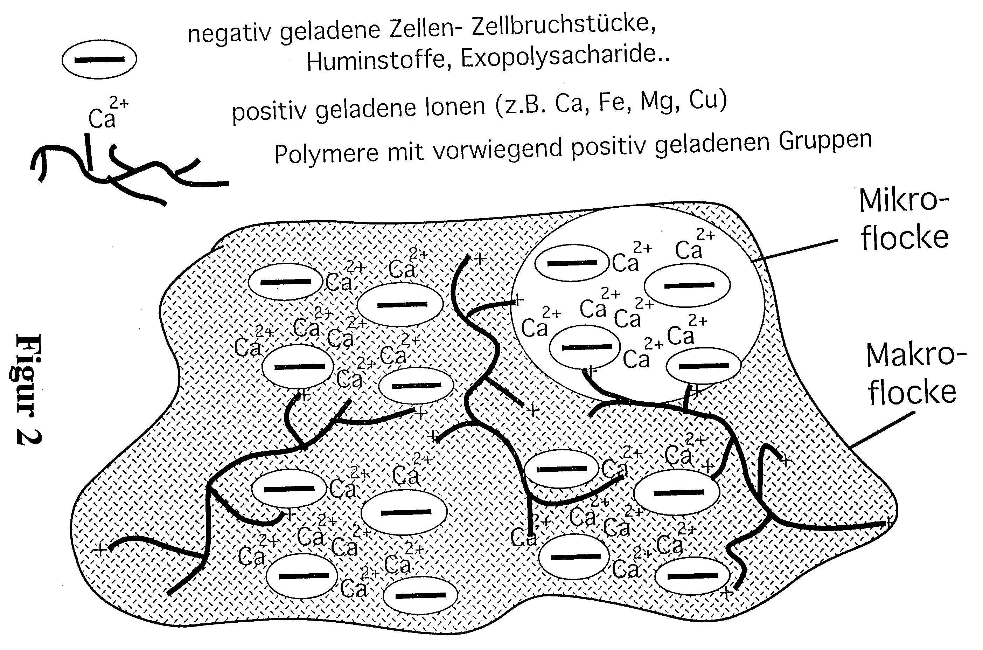

Es ist dabei durchaus möglich, daß sich bereits während der aeroben Reinigungsstufe in der Biomasse Flocken bilden, die eine sehr komplexe Form aufweisen. Eine Flocke kann z.B. aus Mikroorganismen, Exopolysacchariden, Huminstoffen, Zellbestandteilen und/oder Sedimentationszusatzstoffen (Polyelektrolyten) bestehen, die mit Hilfe von anorganischen Ionen (z.B. Ca, Mg, Fe, Cu) so komplex gebunden sind, daß sie für die Mikroorganismen nicht zugänglich sind (schematisch in **Figur 2** dargestellt). Ihre Größe dürfte meist im µm- bis mm-Bereich liegen.

Erfindungsgemäß ist es deshalb auch nicht mehr erforderlich und dürfte in den meisten Fällen auch nicht sachdienlich sein, den Schlamm mit Zellaufschlußverfahren vorzubehandeln, da die elektrischen Ladungen bei diesen Verfahren weiterhin im System erhalten bleiben, und nach dem Abschluß der Vorbehandlung die vorherigen Bindungen, selbst wenn sie zwischenzeitlich mechanisch zerstört wurden, sich selbständig wieder stabilisieren. Vielmehr wird vorgeschlagen, vor einem Vergärschritt die Flocken des Klärschlamms physikalisch-chemisch zu zerstören. Bevorzugt werden dabei spezifisch an der Komplexbildung beteiligte Ionen entfernt. Dabei handelt es sich vor allem um Al-, Fe-, Ca- und Mg-Ionen. Sind Schwermetallionen vorhanden, so lassen sich auch diese entfernen. Werden die mehrwertigen Ionen durch einwertige Ionen (z.B. H⁺ oder Na⁺) ersetzt, werden die Flockenstruktur und die Organik in eine Form gebracht, die für die anaerob arbeitenden Mikroorganismen zugänglich ist.

Eine Möglichkeit der physikalisch-chemischen Vorbehandlung ist die, dem Klärschlamm soviel Säure zuzusetzen, bis ein pH-Wert erreicht ist, bei dem die für die Flockenbildung verantwortlichen Ionen ganz oder zum großen Teil aus ihrer komplexgebundenen Form herausgelöst werden und in Lösung gehen. Der bevorzugte pH-Wert hängt von der Zusammensetzung des Schlamms und den vorhandenen Ionen ab und läßt sich vom Fachmann leicht ermitteln. In der Regel dürften pH-Werte im Bereich von 1,5 bis 2,2 geeignet sein, z.B. pH 1,8.

Als Säure eignet sich beispielsweise Salzsäure.

Nach Zugabe der Säure wird die mit den zu entfernenden Ionen angereicherte Flüssigkeit abgetrennt, z.B. durch Abzentrifugieren. Ein Nachwaschen ist möglich, jedoch meist nicht erforderlich und aus Kostengründen nicht zu empfehlen.

Die abgezogene Flüssigkeit wird sodann wieder ergänzt, z.B. durch Zugabe von entionisiertem Wasser. Anschließend wird der pH-Wert in für das Vergären geeigneter Weise eingestellt, vorzugsweise auf einen pH-Wert von ≥6. Dies kann durch Zugabe von Lauge erfolgen. Die Flüssigkeitsergänzung kann statt dessen gleich mit verdünnter Lauge erfolgen, wobei auf die Einstellung des richtigen pH-Wertes und ggf. eine Korrektur derselben zu achten ist.

Feste und flüssige Phase sollten gut durchmischt sein, wenn anschließend ein bzw. der Vergärschritt erfolgt.

Eine weitere, bevorzugte Möglichkeit der physikalisch-chemischen Vorbehandlung ist die, den Klärschlamm mit Ionenaustauscher in Kontakt zu bringen. Als geeignet haben sich dabei schwach saure Kationenaustauscher erwiesen, z.B. Amberlite (Rohm & Haas) mit einer hohen Selektivität für Calcium und Magnesium. Bevorzugt sind die Kationenaustauscher makroporös. Auch stärker bzw. stark saure Austauscher können zum Einsatz kommen, je nach dem Ziel der zu entfernenden Ionenspezies. Eine hohe Selektivität kann durch Chelatgruppen erreicht werden, die chemisch an die Matrix gebunden sind.

Normalerweise wird eine Ionenaustauscher-Behandlung aufgrund reaktionstechnischer Überlegungen (Austauschkinetik) in einer gepackten Säule betrieben. Eine gepackte Säule kann man vom Reaktortyp her als einen Rohrreaktor beschreiben.

Da man den Klärschlamm nicht über eine Säule schicken kann, weil diese sofort verstopft wäre, muß er anders, z.B. in einem Batchbetrieb, behandelt werden. Um wieder eine "Rohrcharakteristik" zu erzielen, kann der Schlamm bevorzugt in zwei oder mehr Stufen behandelt werden (Reaktorkaskade). Der Schlamm sollte dafür mit Ionenaustauscher in Kontakt gebracht, anschließend vom Ionenaustauscher abgetrennt und dann wieder mit frischem Ionenaustauscher in Kontakt gebracht werden. Auf diese Weise ist es möglich, auch mit kleineren Mengen Ionenaustauscher größere Kationenmengen aus dem Schlamm zu entfernen. Das Mengenverhältnis von Ionentauscher zu Schlamm hängt u.a. von der Zusammensetzung des Schlammes, dessen Gehalt und Konzentration an mehrwertigen Ionen und dessen Wassergehalt ab und kann vom Fachmann unschwer ermittelt werden. Übliche Bereiche sind für den Batchbetrieb z.B. Verhältnisse von 5:1 bis 0.1:1, wobei der Ionenaustauscheranteil bei zunehmend guter Rohrcharakteristik abnehmen kann. Gute Ergebnisse erhält man mit einem Verhältnis von Austauscher zu Schlamm von etwa 1:1, sehr gute Ergebnisse erhält man, wenn man ein derartiges Mengenverhältnis in beiden Stufen einer zweistufigen Behandlung einsetzt.

Alternativ kann anstelle eines Batchverfahrens kontinuierlich gearbeitet werden. Zum Beispiel kann Ionenaustauscher dem Schlamm kontinuierlich zugemischt und mittels einer Durchlaufzentrifuge oder eines Hydrozyklons wieder abgetrennt werden.

Wird der Klärschlamm nach einer zweistufigen Vergärung (aerob und anaerob), nachdem schon ein Abbau von ca. 50% erfolgt ist und der Abbau im wesentlichen zum Erliegen gekommen ist, physikalisch-chemisch entflockt (z.B. mit Hilfe von Säure oder Ionenaustauscher), werden die komplexen Strukturen aus Huminstoffen, anorganischen Ionen und Zellen bzw. Zellbruchstücken aufgelöst und zusätzlich der Vergärung zugänglich gemacht. Durch diese Behandlung kann der vorher schon stabilisierte Schlamm erneut vergoren und ein weiterer Abbau der Organik erreicht werden. Die Vergärung des bereits stabilisierten (d.h. ohne die erfinderischen Maßnahmen keiner weiteren Abnahme der Organik zugänglichen) oder aber bis dahin einzig aerob behandelten Schlamms kann einstufig oder mehrstufig, z.B. zweistufig erfolgen.

Durch diese Behandlung kann der Abbau der organischen Bestandteile des Klärschlamms verbessert und somit die Leistungsfähigkeit der Vergärung erheblich gesteigert werden. Der herkömmliche Abbaugrad von 50 - 55% der Organik kann mit Hilfe der oben beschriebenen Behandlung auf über 70 - 80% gesteigert werden.

Die nachfolgenden Beispiele wurden mit Amberlite IRC 718 durchgeführt, zusätzliche Tests erfolgten mit schwach sauren Kationenaustauschern der Firmen Purolite, Duolite, Lewatitt und Relite.

### Beispiel 1 (Batch-Verfahren)

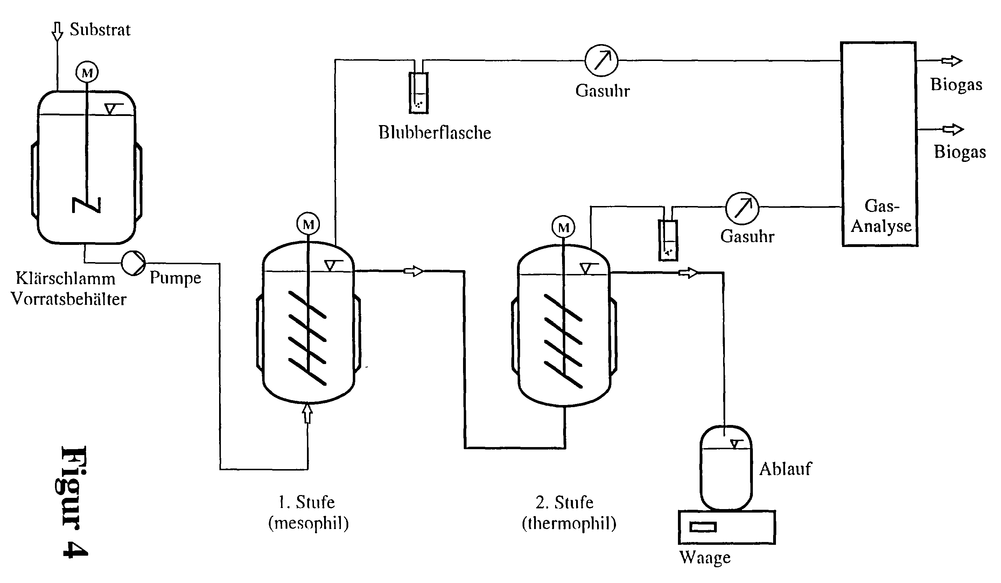

Klärschlamm wurde mit einer 2-stufigen Technikumsanlage, wie in **Figur 4** dargestellt, behandelt. Der dabei erhaltene Schlamm ("stabilisierter Schlamm") besaß noch ca. 50% Restorganik.

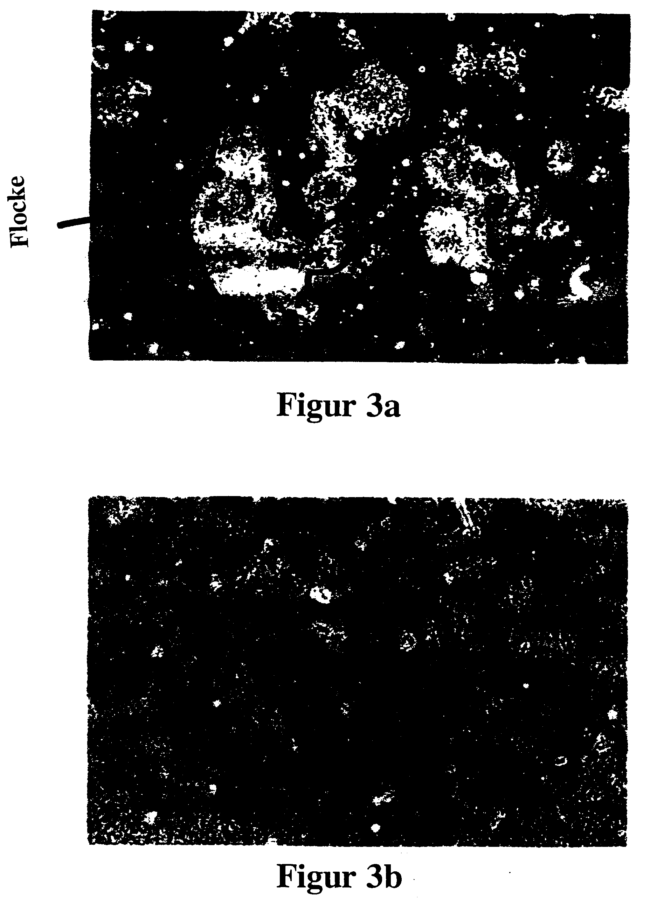

Eine lichtmikroskopische Aufnahme von Schlamm (200-fache Vergrößerung) ist in **Figur 3a** gezeigt. Dieser Schlamm diente als Ausgangssubstrat. Davon wurde ein Wasseranteil (2/3 vom Ausgangsvolumen) in einer Zentrifuge abgetrennt, und die Trockensubstanz wurde im Restwasser durch Rühren wieder gleichmäßig verteilt. Danach waren 18 l Schlamm mit einer Konzentration der Organik von 30 g/l übrig. Dieser Schlamm wurde in drei gleich große Chargen A, B und C aufgeteilt. Bis zu diesem Zeitpunkt waren alle drei Chargen identisch.

Anschließend wurden A und B mit Ionenaustauscher behandelt. Charge C blieb unbehandelt. Bei der Ionenaustauscherbehandlung wurden jeweils 6 l Schlamm und 6 l Ionenaustauscher in ein Gefäß gegeben. Mit einem Rührer wurde diese Mischung 1 Stunde lang gerührt (Batch-Betrieb). Nach der Ionenaustauscherbehandlung wurde mit einer Zentrifuge der Ionenaustauscher vom Schlamm abgetrennt. Eine lichtmikroskopische Aufnahme des Schlamms nach der Ionenaustauscherbehandlung, im selben Maßstab wie die Aufnahme der Figur 3a, ist in **Figur 3b** dargestellt. Man erkennt deutlich, daß die hell erscheinenden, großen Flocken (siehe umrahmte "Flocke") nicht mehr vorhanden sind. Es ist davon auszugehen, daß bei der Behandlung ca. 1/3 der ursprünglich im Schlamm enthaltenen Kationen aus dem Schlamm entfernt wurden.

Diese drei nun bereitgestellten Chargen wurden unter gleichen Bedingungen wiederum einer 2-stufigen Vergärung zugeführt. Bei der ersten Stufe handelte es sich um ein 1,6 l, bei der zweiten Stufe um ein 0,8 l Reaktor. Die Vergärung wurde kontinuierlich betrieben. Es wurde 1 mal täglich eine kleine, gleichbleibende Menge an Schlamm aus den Reaktoren entnommen und aus den vorbereiteten Chargen eine entsprechende Menge neuer Schlamm den Reaktoren zugegeben. Die Reaktoren wurden mesophil (37°C) und mit einer Verweilzeit von 16 Tagen betrieben.

Durch die Messung der oTS (organische Trockensubstanz) vor und nach den Reaktoren kann der Umsatz in % ermittelt werden. Dazu wurden 3 mal pro Woche Proben aus den Reaktoren entnommen und analysiert.

### Ergebnis:

Bei den Chargen A und B (behandelt) stellte sich ein Umsatz von 40%, bei der Charge (C (unbehandelt) ein Umsatz von 18% ein. (Anmerkung: Auch "stabilisierter Schlamm", wie für Charge C verwendet, wird bei längeren Verweilzeiten geringfügig weiter abgebaut, da er anaerobe Biomasse und sehr langsam abbaubare Substanzklassen enthält.)

Berücksichtigt man, daß der Schlamm in der Technikumsanlage schon zu 50% abgebaut worden war, ergibt sich ein Gesamtumsatz für A und B von 70%, für C von 59%.

### Beispiel 2 (kontinuierliches Verfahren)

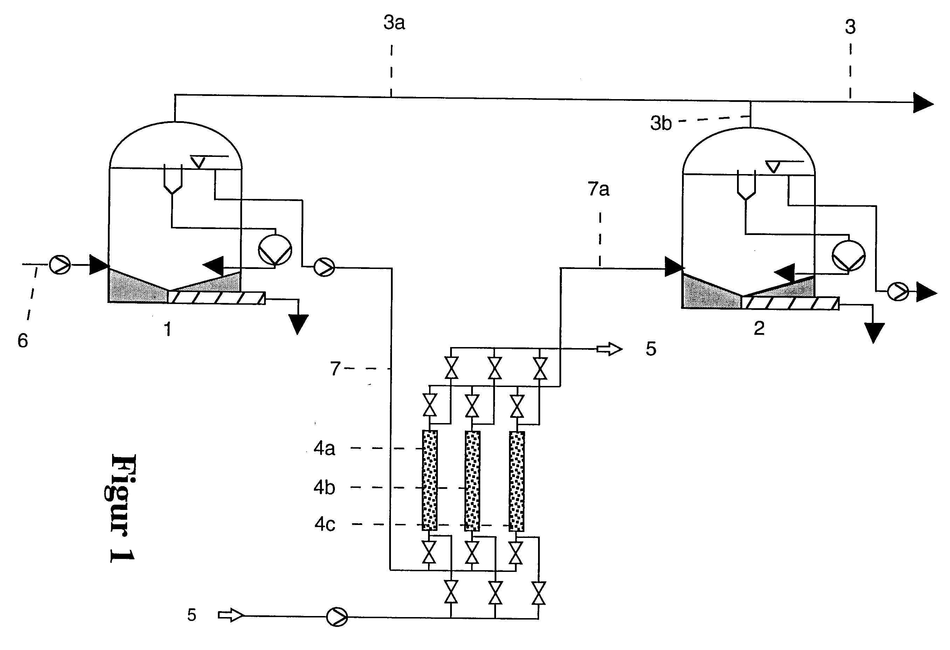

Die Behandlung mit Ionenaustauscher (ggf. eine andere Entflockungsmethode) erfolgt hier in Wirbelbetten, die von unten angeströmt werden. Alternativen sind Hydrozyklone oder Durchlaufzentrifugen. Die Behandlung des Klärschlamms kann wahlweise vor einer 2-stufigen Vergärung oder, wie in **Figur 1** dargestellt, zwischen der ersten und der zweiten anaeroben Stufe erfolgen. **In Figur 1** erkennt man einen ersten Faulturm/Klärschlammbehälter 1, in den über die Leitung 6 der Klärschlamm eingebracht wird. Die während der anaeroben Vergärung entstehenden Faulgase (vor allem CO₂ und Methan) können über die Leitungen 3a und 3 abgezogen werden. Nach Abschluß der Vergärung wird der Schlamm über die Leitung 7 einer Vorrichtung zum Entflocken 4a, 4b, 4c zugeführt, die hier schematisch in Form dreier Rohrreaktoren dargestellt ist. Dabei sollte der Schlamm in einem oder mehreren der Reaktoren (4a,4b) behandelt werden, während der oder die nicht benutzten Reaktoren (4c) über eine Spül- und Regenerationsmittel-Leitung 5 gespült und ggf. wieder aktiviert werden. Der entflockte Schlamm wird über die Leitung 7a dem Faulturm/Klärschlammbehälter 2 zugeführt, wo er nun nochmals anaerob vergoren wird.

Bei einer solchen Behandlung zwischen den zwei Vergärstufen ist eine vorherige Aufkonzentrierung des Schlamms durch Wasserentfernung möglich (siehe z.B. Beispiel 1). Während der eigentlichen Behandlung wird der Schlamm solange mit einem sauren Ionenaustauscher in Kontakt gebracht, bis die mehrwertigen Kationen (vor allem vorzugsweise Ca-Ionen) aus dem Schlamm entfernt sind. Durch Aufsummieren der Abbauraten vom Eintritt in die erste bis zum Verlassen der zweiten Vergärungsstufe wird ein Abbau der organischen Bestandteile von > 80% erzielt.

## Patentansprüche

1. Verfahren zum anaeroben Vergären von Klärschlamm, **dadurch gekennzeichnet**, daß der Klärschlamm physikalisch-chemisch entflockt wird.

2. Verfahren nach Anspruch 1, **dadurch gekennzeichnet**, daß dem Klärschlamm flockenbildende Ionen entzogen werden.

3. Verfahren nach Anspruch 2, **dadurch gekennzeichnet**, daß der Klärschlamm mit Ionenaustauscher in Kontakt gebracht wird.

4. Verfahren nach Anspruch 2, **dadurch gekennzeichnet**, daß dem Klärschlamm soviel Säure zugegeben wird, daß zumindest ein großer Teil der flockenbildenden Ionen in Lösung geht, die gelöste Phase abgetrennt und anschließend zum Vergären der pH-Wert auf ≥ 6 eingestellt wird.

5. Verfahren nach Anspruch 4, **dadurch gekennzeichnet**, daß mit der Säurezugabe ein pH-Wert von 1,6 bis 2,0 eingestellt wird.

6. Verfahren nach einem der voranstehenden Ansprüche, **dadurch gekennzeichnet**, daß vor einem Vergärschritt der Klärschlamm entflockt wird.

7. Verfahren nach Anspruch 6, **dadurch gekennzeichnet**, daß vor dem ersten Vergärschritt der Klärschlamm entflockt wird.

8. Verfahren nach Anspruch 6, **dadurch gekennzeichnet**, daß nach einem ersten Vergärschritt der Klärschlamm entflockt wird.

9. Verfahren nach Anspruch 3, **dadurch gekennzeichnet**, daß während des Vergärens oder während eines Vergärschritts dem Schlamm Ionenaustauscher zugesetzt und anschließend wieder davon abgetrennt wird.
